# EUROPÄISCHE PATENTSCHRIFT

(11) **EP 0 432 694 B1**
(45) Veröffentlichungstag und Bekanntmachung des Hinweises auf die Patenterteilung: **29.05.1996**
(21) Anmeldenummer: 90123696.8
(22) Anmeldetag: 10.12.1990
(51) Int. Cl.: C07D 217/26, A61K 31/47, C07D 303/36, C07D 401/06, C07D 405/06, C07D 497/10, C07D 209/48, C07C 247/02

(54) **Aminoalkohole**
Aminoalcohols
Aminoalcools

(30) Priorität: 11.12.1989 GB 8927915
(43) Veröffentlichungstag der Anmeldung: 19.06.1991
(60) Teilanmeldung: 95102405.8
(73) Patentinhaber: F. HOFFMANN-LA ROCHE AG, CH-4002 Basel (CH)
(72) Erfinder: Parkes, Kevin Edward Burdon, Letchworth, Herts (GB); Redshaw, Sally, Stevenage, Herts (GB); Thomas, Gareth John, Oaklands, Welwyn, Herts (GB)
(74) Vertreter: Lederer, Franz, Dr.

(56) Entgegenhaltungen:
- TETRAHEDRON LETTERS, Band 25, Nr. 51, 1984, Seiten 5899-5902, Pergamon Press Ltd, GB; M. BESSODES et al.: "Enantiospecific synthesis of the immunopotentiatiors erythro-9 (2-hydroxy-3-nonyl) hypoxanthines and the threo-diastereomers"
- JUSTUS LIEBIGS ANNALEN DER CHEMIE, 1988, Seiten 445-448, Verlagsgesellschaft mbH, Weinheim, DE; J. MULZER et al.: "Synthesis of diastereomerically and enantiomerically pure (3S,4S)-statine from (R)-2,3-O-isopropylideneglyceraldehyde"
- JOURNAL OF ORGANIC CHEMISTRY, Band 52, Nr. 8, August 1987, pages 1487-1492, American Chemical Society, Washington DC, US; J.R. LULY et al.: "A synthesis of protected aminoalkyl epoxides from alpha-amino acids"

## Beschreibung

Die vorliegende Erfindung betrifft neue Alkohole. Insbesondere betrifft sie neue Alkohole, ein Verfahren zu deren Herstellung sowie neue Zwischenprodukte in dem genannten Verfahren.

Die erfindungsgemässen neuen Alkohole sind Verbindungen der allgemeinen Formel 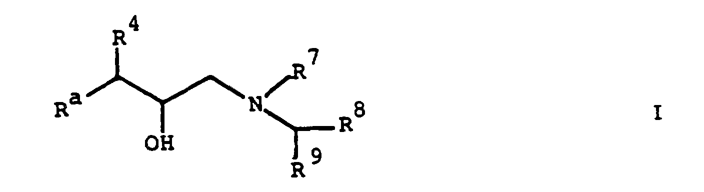 worin R^{a} Azido oder Phthalimido, R⁴ Alkyl, Cycloalkyl, Cycloalkylalkyl, Aryl oder Aralkyl und R⁷ und R⁸ zusammen eine Trimethylen- oder Tetramethylengruppe bedeuten, welche gegebenenfalls durch Hydroxy, Alkoxycarbonylamino oder Acylamino substituiert ist oder in welcher eine -CH₂-Gruppe durch -NH-, -N(Alkoxycarbonyl)-, -N(Acyl)- oder -S- ersetzt ist oder welche einen anellierten Cycloalkan-, aromatischen oder heteroaromatischen Ring trägt, und R⁹ Alkoxycarbonyl, Monoalkylcarbamoyl, Monoaralkylcarbamoyl, Monoarylcarbamoyl oder eine Gruppe der Formel 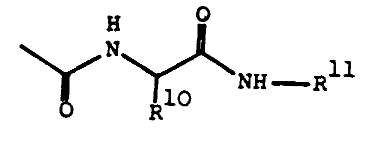 bedeutet, in welcher R¹⁰ und R¹¹ je Alkyl bedeuten.

Die Alkohole der Formel I sind wertvolle Zwischenprodukte in chemischen Synthesen. Beispielsweise können sie in der weiter unten genauer beschriebenen Weise in Aminosäurederivate der nachstehenden Formel übergeführt werden 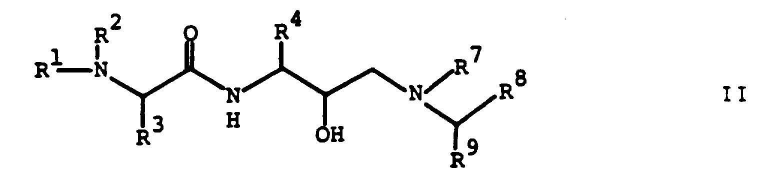 worin R¹ Alkoxycarbonyl, Aralkoxycarbonyl, Alkanoyl, Cycloalkylcarbonyl, Aralkanoyl, Aroyl, Heterocydylcarbonyl, Alkylsulfonyl, Arylsulfonyl, Monoaralkylcarbamoyl, Cinnamoyl oder α-Aralkoxycarbonylaminoalkanoyl und R Wasserstoff oder R¹ und R zusammen mit dem Stickstoffatom, an welches sie gebunden sind, eine cyclische Imidgruppe der Formel 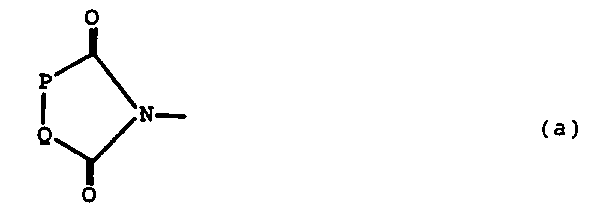 in welcher P und Q zusammen ein aromatisches System bedeuten, und R³ Alkyl, Cydoalkyl, Aryl, Aralkyl, Heterocydylalkyl, Cyanoalkyl, Alkylthioalkyl, Alkylsulfinylalkyl, Carbamoylalkyl oder Alkoxycarbonylalkyl bedeuten und R⁴, R⁷, R⁸ und R⁹ die oben angegebene Bedeutung besitzen.

Diese Aminosäurederivate besitzen wertvolle pharmakologische Eigenschaften. Insbesondere hemmen sie Proteasen viralen Ursprungs und können zur Prophylaxe oder Behandlung viraler Infektionen verwendet werden, insbesondere solcher Infektionen, die durch HIV und andere Retroviren verursacht werden ( vgl. Britische Patentanmeldung Nr. 8908035).

Der in der vorliegenden Beschreibung verwendete Ausdruck "Alkyl" - allein oder in Kombination - betrifft geradkettige oder verzweigte Alkylreste mit maximal 8, vorzugsweise maximal 4, Kohlenstoffatomen, wie Methyl, Aethyl, n-Propyl, Isopropyl, n-Butyl, Isobutyl, sec.Butyl, tert.Butyl, Pentyl, Hexyl und dergleichen. Der Ausdruck "Alkoxy" - allein oder in Kombination - betrifft eine Alkyläthergruppe, in welcher der Ausdruck "Alkyl" die oben angegebene Bedeutung besitzt, wie Methoxy, Aethoxy, n-Propoxy, Isopropoxy, n-Butoxy, Isobutoxy, sec.Butoxy, tert.Butoxy und dergleichen. Der Ausdruck "Cycloalkylalkyl" betrifft eine weiter oben definierte Alkylgruppe, welche durch eine Cycloalkylgruppe mit 3-8, vorzugsweise 3-6, Kohlenstoffatomen, wie Cydopropyl, Cyclobutyl, Cydopentyl, Cydohexyl und dergleichen, substituiert ist. Der Ausdruck "Aryl" - allein oder in Kombination - betrifft eine Phenyl- oder Naphthylgruppe, welche durch Alkyl, Alkoxy, Halogen, Hydroxy, Amino und dergleichen ein- oder mehrfach substituiert sein kann, wie Phenyl, p-Tolyl, 4-Methoxyphenyl, 4-tert.Butoxyphenyl, 4-Fluorphenyl, 4-Chlorphenyl, 4-Hydroxyphenyl, 1-Naphthyl, 2-Naphthyl etc. Der Ausdruck "Aralkyl" - allein oder in Kombination - betrifft eine Alkylgruppe wie sie oben definiert ist, in der ein Wasserstoffatom durch eine weiter oben definierte Arylgruppe ersetzt ist, wie Benzyl, 2-Phenyläthyl und dergleichen. Der Ausdruck "Aralkoxycarbonyl" - allein oder in Kombination - betrifft eine Gruppe der Formel -C(O)-O-Aralkyl, in welcher der Ausdruck "Aralkyl" die oben angegebene Bedeutung besitzt, wie Benzyloxycarbonyl etc. Der Ausdruck "Alkanoyl" - allein oder in Kombination - betrifft eine Acylgruppe, welche von einer Alkancarbonsäure abgeleitet ist, wie Acetyl, Propionyl, Butyryl, Valeryl, 4-Methylvaleryl etc. Der Ausdruck "Cycloalkylcarbonyl" betrifft eine Acylgruppe, welche von einer monocyclischen oder verbrückten Cycloalkancarbonsäure abgeleitet ist, wie Cyclopropancarbonyl, Cyclohexancarbonyl, Adamantancarbonyl etc., oder welche von einer benz-anellierten monocyclischen, gegebenenfalls durch beispielsweise Alkanoylamino substituierten Cydoalkancarbonsäure abgeleitet ist, wie 1,2,3,4-Tetrahydro-2-naphthoyl, 2-Acetamido-1,2,3,4-tetrahydro-2-naphthoyl. Der Ausdruck "Aralkanoyl" betrtifft eine Acylgruppe, welche von einer Aryl- substituierten Alkancarbonsäure abgeleitet ist, wie Phenylacetyl, 3-Phenylpropionyl(hydrocinnamoyl), 4-Phenylbutyryl, (2-Naphthyl)acetyl, 4-Chlorhydrocinnamoyl, 4-Aminohydrocinnamoyl, 4-Methoxyhydrocinnamoyl etc. Der Ausdruck "Aroyl" betrifft eine Acylgruppe, welche von einer aromatischen Carbonsäure abgeleitet ist, beispielsweise einer gegebenenfalls substituierten Benzoe- oder Naphthoesäure, wie Benzoyl, 4-Chlorbenzoyl, 4-Carboxybenzoyl, 4-(Benzyloxycarbonyl)benzoyl, 1-Naphthoyl, 2-Naphthoyl, 6-Carboxy-2-naphthoyl, 6-(Benzyloxycarbonyl)-2-naphthoyl, 3-Benzyloxy-2-naphthoyl, 3-Hydroxy-2-naphthoyl, 3-(Benzyloxyformamido)-2-naphthoyl etc. Der Heterocyclylteil einer Heterocyclylcarbonyl- oder Heterocyclylalkylgruppe ist ein gesättigter, teilweise ungesättigter oder aromatischer monocyclischer, bicyclischer oder tricyclischer Heterocydus, welcher ein oder mehrere Stickstoff-, Sauerstoff- oder Schwefelatome als Heteroatome enthält und welcher gegebenenfalls an einem oder mehreren Kohlenstoffatomen durch Halogen, Alkyl, Alkoxy, Oxo etc. und/oder an einem sekundären Stickstoffatom (d.h. -NH-) durch Alkyl, Aralkoxycarbonyl, Alkanoyl, Phenyl oder Phenylalkyl oder an einem tertiären Stickstoffatom (d.h. =N-) durch Oxido substituiert ist und über ein Kohlenstoffatom gebunden ist. Beispiele solcher Heterocydylgruppen sind Pyrrolidinyl, Piperidinyl, Piperazinyl, Morpholinyl, Thiamorpholinyl, Pyrrolyl, Imidazolyl, beispielsweise Imidazol-4-yl, 1-Benzyloxycarbonylimidazol-4-yl etc., Pyrazolyl, Pyridyl, Pyrazinyl, Pyrimidinyl, Furyl, Thienyl, Triazolyl, Oxazolyl, Thiazolyl, Indolyl, beispielsweise 2-Indolyl etc., Chinolyl, beispielsweise 2-Chinolyl, 3-Chinolyl, 1-Oxido-2-chinolyl etc., Isochinolyl, beispielsweise 1-Isochinolyl, 3-Isochinolyl etc., Tetrahydrochinolyl, beispielsweise 1,2,3,4-Tetrahydro-2-chinolyl etc., 1,2,3,4-Tetrahydroisochinolyl, beispielsweise 1,2,3,4-Tetrahydro-1-oxo-isochinolyl etc., Chinoxalinyl, β-Carbolinyl und dergleichen. Der Ausdruck "Halogen" betrifft Fluor, Chlor, Brom und Jod.

Eine Cinnamoylgruppe R¹ kann unsubstituiert sein oder am Phenylring einen oder mehrere Substituenten aus Alkyl, Alkoxy, Halogen, Nitro und dergleichen tragen.

Das durch P und Q bezeichnete aromatische System in der oben angegebenen Formel (a) kann monocyclisch, beispielsweise 1,2-Phenylen oder Thienylen, oder polycyclisch, beispielsweise 1,2-Naphthylen, 2,3-Naphthylen, 1,8-Naphthylen, 2,3-Anthrylen etc., und unsubstituiert oder durch einen oder mehrere Substituenten aus Alkyl, Alkoxy, Halogen und dergleichen substituiert sein.

Wie oben erwähnt kann eine durch R⁷ und R⁸ bezeichnete Trimethylen- oder Tetramethylengruppe durch eine Hydroxygruppe, eine Alkoxycarbonylaminogruppe, beispielsweise tert.Butoxycarbonylamino, oder eine Acylaminogruppe, d.h. eine Alkanoylamino-, Cycloalkylcarbonylamino-, Aralkanoylamino- oder Aroylaminogruppe, substituiert sein. Alternativ kann auch eine -CH₂-Gruppe der mit R⁷ und R⁸ bezeichneten Trimethylen- oder Tetramethylengruppe durch -NH-, -N(Alkoxycarbonyl)-, beispielsweise -N(tert.Butoxycarbonyl)-, -N(Acyl)- oder -S- ersetzt sein. Wenn eine durch R⁷ und R⁸ bezeichnete Trimethylen- oder Tetramethylengruppe einen anellierten Cycloalkanring trägt, so kann dieser beispielsweise ein Cycloalkanring mit 3-6 Kohlenstoffatomen, wie Cyclopentan, Cyclohexan oder dergleichen, sein, und wenn die Trimethylen- oder Tetramethylengruppe einen anellierten aromatischen oder heteroaromatischen Ring trägt, so kann dieser beispielsweise ein Berzol-, Indol- oder Thiophenring sein, welcher gegebenenfalls an einem oder mehreren Kohlenstoffatomen durch Halogen, Alkyl, Alkoxy etc. substituiert sein kann. So kann -N(R⁷)-CH(R⁸)(R⁹) beispielsweise eine der folgenden Gruppen bedeuten: 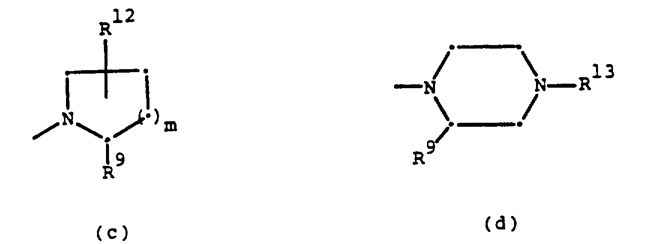 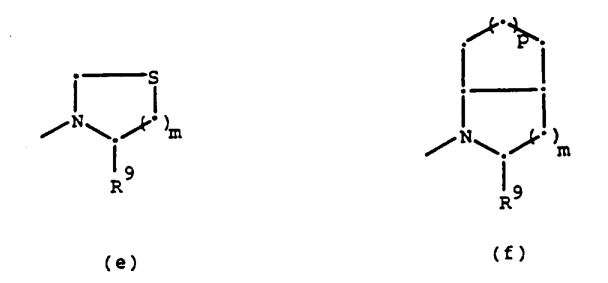 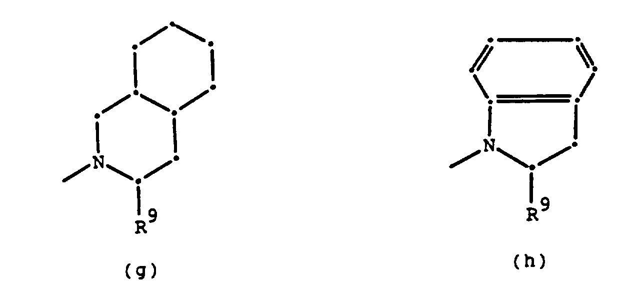 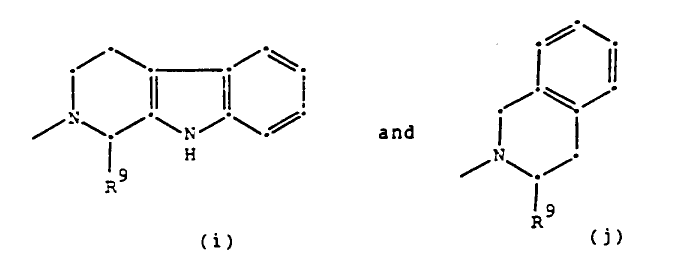 worin R⁹ die oben angegebene Bedeutung besitzt, R¹ Wasserstoff, Hydroxy, Alkoxycarbonylamino oder Acylamino, R¹³ Wasserstoff, Alkoxycarbonyl oder Acyl und m und p je 1 oder 2 bedeuten.

Die Verbindungen der Formel I besitzen mindestens zwei asymmetrische Kohlenstoffatome und können deshalb in Form von optisch reinen Diastereomeren, Gemischen von Diastereomeren, diastereomeren Racematen oder Gemischen von diastereomeren Racematen vorliegen. Die vorliegende Erfindung umfasst alle diese Formen.

In der obigen Formel I bedeutet R^{a} vorzugsweise Phthalimido. R⁴ bedeutet vorzugsweise Aralkyl, insbesondere Benzyl. -N(R⁷)-CH(R⁸)(R⁹) bedeutet vorzugsweise eine Gruppe der obigen Formel (g), worin R⁹ Monoalkylcarbamoyl bedeutet, insbesondere tert.Butylcarbamoyl.

Aus dem obigen folgt, dass besondere bevorzugte Verbindungen der Formel I solche sind, worin R^{a} Phthalimido, R⁴ Benzyl und -N(R⁷)-CH(R⁸)(R⁹) eine Gruppe der Formel (g) bedeuten, worin R⁹ tert.Butylcarbamoyl bedeutet.

N-tert.Butyl-decahydro-2-[2(R)-hydroxy-4-penyl-3(S)-phthalimidobutyl](4aS,8aS)-isochinolin-3(S)-carboxamid ist ein ganz besonders bevorzugter Alkohol der obigen Formel I.

2-[3(S)-Azido-2(R)-hydroxy-4-phenylbutyl]-N-tert.butyl-decahydro(4aS,8aS)-isochinolin-3(S)-carboxamid ist ein weiteres Beispiel einer Verbindung der obigen Formel I.

Die Alkohole der obigen Formel I können erfindungsgemäss hergestellt werden, indem man
(a) ein Epoxid der allgemeinen Formel 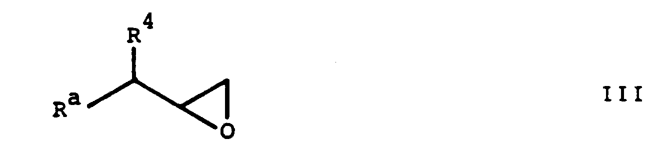 worin R^{a} und R⁴ die oben angegebene Bedeutung besitzen, mit einer Verbindung der allgemeinen Formel 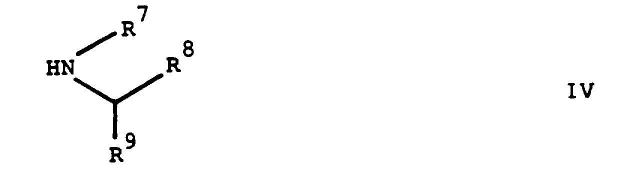 worin R⁷, R⁸ und R⁹ die oben angegebene Bedeutung besitzen, umsetzt, oder
(b) zur Herstellung eines Alkohols der Formel I, worin R^{a} Azido bedeutet, eine Verbindung der allgemeinen Formel 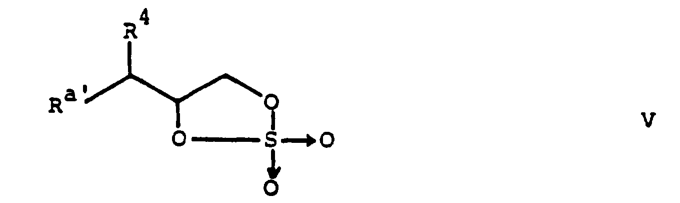 worin R^{a'}Azido bedeutet und R⁴ die oben angegebene Bedeutung besitzt, mit einer Verbindung der obigen Formel IV umsetzt.

Die Umsetzung eines Epoxids der Formel III mit einer Verbindung der Formel IV gemäss Verfahrensvariante (a) wird zweckmässig in einem inerten organischen Lösungsmittel, wie einem Alkanol, z.B. Methanol etc., Dimethylformamid oder dergleichen und erhöhter Temperatur durchgeführt, zweckmässig bei ungefähr 60°C bis ungefähr 120°C.

Die Umsetzung einer Verbindung der Formel V mit einer Verbindung der Formel IV gemäss Verfahrensvariante (b) wird zweckmässig in einem inerten organischen Lösungsmittel, wie einem Aether, z.B. Tetrahydrofuran etc., und bei ungefähr Raumtemperatur durchgeführt.

Die Epoxide der Formel III, worin R⁴ Hexyl worin R^{a} Azido ist und worin R⁴ Isobutyl worin R^{a} Phthalimido ist, sind bekannt.

Die restlichen in Verfahrensvariante (a) als Ausgangsstoffe eingesetzten Epoxide der Formel III sind neu und ebenfalls Gegenstand der vorliegenden Erfindung. Die Epoxide der Formel III, worin R^{a} Azido bedeutet, können hergestellt werden, indem man beispielsweise eine Verbindung der allgemeinen Formel 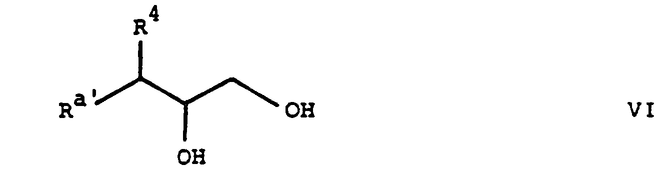 worin R^{a'} und R⁴ die obige Bedeutung besitzen, in bekannter Weise in eine Verbindung der allgemeinen Formel 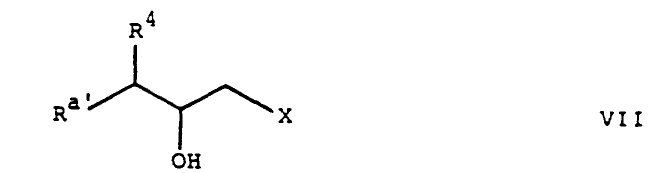 worin R^{a'} und R⁴ die oben angegebene Bedeutung besitzen und X eine Abgangsgruppe, wie ein Halogenatom, z.B. Chlor oder Brom, eine Alkansulfonyloxygruppe, z.B. Methansulfonyloxy etc., oder eine aromatische Sulfonyloxygruppe, z.B. Benzolsulfonyloxy, p-Toluolsulfonyloxy, 2,4,6-Triisopropylbenzolsulfonyloxy etc., bedeutet,
überführt und die erhaltene Verbindung der Formel VII in ebenfalls bekannter Weise, beispielsweise durch Behandeln mit einem Alkalimetallhydroxid in einem Alkanol, wie Kaliumhydroxid in Aethanol, zum erwünschten Epoxid der Formel m cyclisiert.

Die Verbindungen der Formel VII sind neu. Andererseits sind die Verbindungen der Formel VI bekannt oder Analoga davon, welche in Analogie zur Herstellung der bekanten Verbindungen hergestellt werden können. Darüberhinaus enthalten die nachfolgenden Beispiele eine detaillierte Beschreibung einer alternativen Methode zur Herstellung gewisser Verbindungen der Formel VI.

Die Epoxide der Formel III, worin R^{a} Phthalimido bedeutet, können beispielsweise hergestellt werden wie dies im nachstehenden Reaktionsschema I veranschaulicht ist, in welchem R^{a"} Phthalimido bedeutet, R⁴ die oben angegebene Bedeutung besitzt und Y Alkylsulfonyl und Z' eine durch Hydrolyse entfernbare Hydroxyschutzgruppe, beispielsweise Tetrahydropyranyl, bedeuten.

### Reaktionsschema I

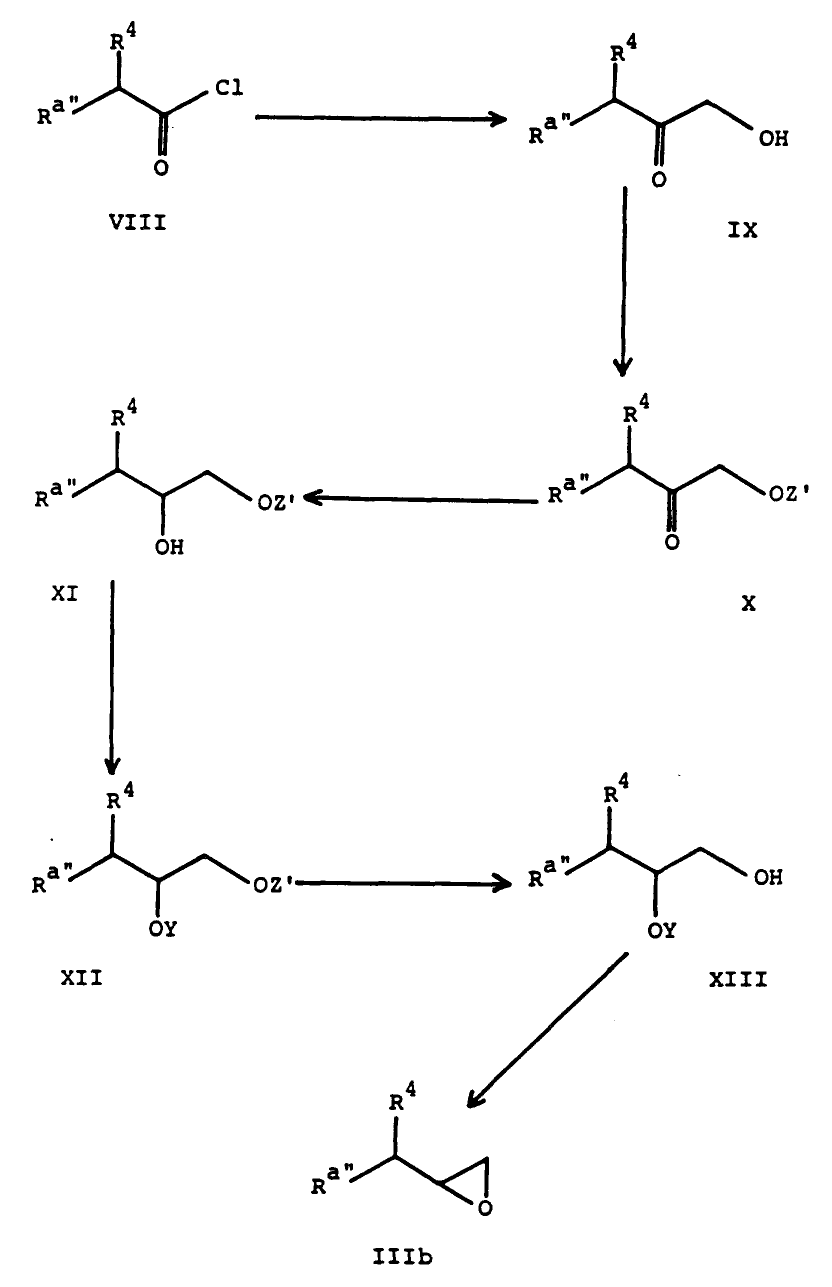

Im ersten Schritt des Reaktionsschema I wird eine Verbindung der Formel VIII mit einem Tris(trialkylsilyloxy)äthylen, wie Tris(trimethylsilyloxy)äthylen, zweckmässig bei erhöhter Temperatur, wie etwa 90°C bis ungefähr 100°C, zu einer Verbindung der Formel IX umgesetzt. Diese Verbindung wird dann an der Hydroxygruppe in bekannter Weise geschützt, beispielsweise durch Umsetzen mit Dihydropyran, und die erhaltene Verbindung der Formel X wird dann in ebenfalls bekannter Weise an der Carbonylgruppe reduziert, beispielsweise mit Hilfe eines komplexen Metallhydrids, z.B. Natriumborhydrid etc. Die erhaltene Verbindung der Formel XI wird dann durch Umsetzen mit einem Alkansulfonylhalogenid, z.B. Methansulfonylchlorid, gemäss bekannten Methoden in eine Verbindung der Formel XII übergeführt, welche ihrerseits in bekannter Weise, z.B. mittels p-Toluolsulfonsäure, zu einer Verbindung der Formel XIII hydrolysiert wird. Eine Verbindung der Formel XIII wird dann schliesslich in ein Epoxid der Formel IIIb übergeführt, indem man diese mit einer Base wie einem Alkalimetallhydrid, z.B. Natriumhydrid, oder einem Alkalimetall-nieder-alkoxid, z.B. Kalium-tert.butoxid, in einem inerten organischen Lösungsmittel, wie Dimethylformamid etc., umsetzt.

Die Verbindungen der obigen Formel VIII sind bekannte Verbindungen oder Analoga davon, welche in ähnlicher Weise zur Herstellung der bekannten Verbindungen erhalten werden können, während die Verbindungen der Formeln IX bis XIII neu sind.

Die in den Verfahrensvarianten (a) und (b) als Ausgangsstoffe eingesetzten Verbindungen der Formel IV sind bekannte Verbindungen oder können leicht aus bekannten Verbindungen hergestellt werden, beispielsweise durch Ueberführen der Carboxylgruppe in einer entsprechenden Carbonsäure in die Gruppe R⁹.

Die in Verfahrensvariante (b) als Ausgangsstoffe verwendeten Verbindungen der Formel V sind neu und ebenfalls Gegenstand der vorliegenden Erfindung. Sie können beispielsweise hergestellt werden, indem man eine Verbindung der Formel VI mit Thionylchlorid umsetzt, wobei die Reaktion zweckmässig in einem inerten organischen Lösungsmittel, wie einem halogenierten Kohlenwasserstoff, z.B. Tetrachlorkohlenstoff etc., und bei erhöhter Temperatur, vorzugsweise bei der Rückflusstemperatur des Reaktionsgemisches, durchgeführt wird, und das Reaktionsprodukt in bekannter Weise oxidiert. Die Oxidation wird zweckmässig mit Ruthenium(III)chlorid in Gegenwart eines Alkalimetallperjodates, z.B. Natriummetaperjodat, bei ungefähr Raumtemperatur durchgeführt. Das Reaktionsprodukt wird zweckmässig in situ oxidiert.

Wie bereits erwähnt können die Alkohole der Formel I in Aminosäurederivate der Formel II übergeführt werden, welche wertvolle pharmakologische Eigenschaften besitzen. Diese Ueberführvng kann beispielsweise so durchgeführt werden, dass man die Phthalimido- oder Azidogruppe R^{a} im Alkohol in eine Aminogruppe überführt und die erhaltene Verbindung der allgemeinen Formel 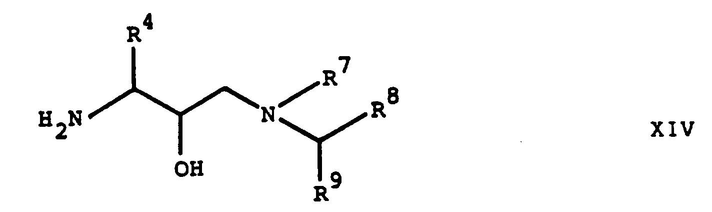 worin R⁴, R⁷, R⁸ und R⁹ die oben angegebene Bedeutung besitzen, mit einer Säure der allgemeinen Formel 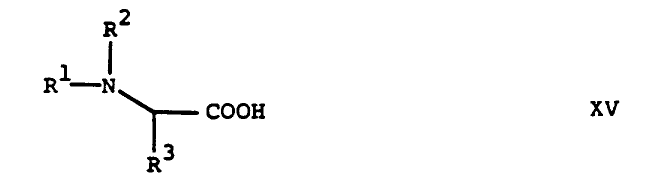 worin R¹, R und R³ die oben angegebene Bedeutung besitzen, oder einem reaktiven Derivat davon umsetzt.

Die Ueberführung der Phthalimido- oder Azidogruppe R^{a} in einem Alkohol der Formel I in eine Aminogruppe kann nach bekannten Methoden erfolgen. So kann die Phthalimidogruppe durch Behandeln mit Hydrazin oder einem primären Amin, z.B. einem Alkylamin, wie Methylamin, und die Azidogruppe durch katalytische Hydrierung in die Aminogruppe übergeführt werden.

Die Umsetzung einer Verbindung der Formel XIV mit einer Säure der Formel XV oder einem reaktiven Derivat davon kann nach in der Peptidchemie bekannten Methoden durchgeführt werden. So wird, falls eine Säure der Formel XV verwendet wird, die Reaktion vorzugsweise in Gegenwart eines Kondensationsmittels, wie Hydroxybenzotriazol und Dicydohexylcarbodiimid, durchgeführt. Diese Reaktion erfolgt zweckmässig in einem inerten organischen Lösungsmittel, wie einem Aether, z.B. Diäthyläther, Tetrahydrofuran etc., oder Dimethylformamid, bei tiefer Temperatur, zweckmässig bei ungefähr -10°C bis ungefähr +5°C, insbesondere bei ungefähr 0°C. Geeignete reaktive Derivate der Säuren der Formel XV, welche verwendet werden können, sind beispielsweise die entsprechenden Säurehalogenide, z.B. Säurechloride, Säureanhydride, gemischte Anhydride, aktivierte Ester etc. Wenn ein reaktives Derivat verwendet wird, erfolgt die Reaktion zweckmässig in einem inerten organischen Lösungsmittel, wie einem halogenierten aliphatischen Kohlenwasserstoff, z.B. Dichlormethan etc., oder einem Aether, z.B. Diäthyläther, Tetrahydrofuran etc., und, wo angezeigt, in Gegenwart einer organischen Base, z.B. N-Aethylmorpholin, Diisopropyläthylamin etc., bei tiefer Temperatur, zweckmässig bei ungefähr -10°C bis +5°C, insbesondere bei ungefähr 0°C.

Die Säuren der Formel XV und deren reaktive Derivate, soweit es nicht bekannte Verbindungen oder Analoga der bekannten Verbindungen sind, können in ähnlicher Weise wie die bekannten Verbindungen hergestellt werden.

Die folgenden Beispiele veranschaulichen die Herstellung der Alkohole der Formel I.

### Beispiel 1

Eine Lösung von 4,39 g (15 mMol) 2(S)-[2-Phenyl-1(S)-phthalimidoäthyl]oxiran und 3,57 g (15 mMol) N-tert.Butyl-decahydro-(4aS,8aS)-isochinolin-3(S)-carboxamid in 30 ml Dimethylformamid wurde unter Rühren 10 Stunden auf 120°C erhitzt und danach 18 Stunden bei Raumtemperatur stehengelassen. Das Reaktionsgemisch wurde unter vermindertem Druck zur Trockene eingedampft. Der Rückstand wurde in 50 ml Aethylacetat gelöst, mit 30 ml Wasser gewaschen, über wasserfreiem Natriumsulfat getrocknet, filtriert und eingedampft. Das Rohprodukt wurde aus Aethylacetat/n-Hexan kristallisiert, wobei 4,77 g N-tert.Butyl-decahydro-2-[2(R)-hydroxy-4-phenyl-3(S)-phthalimidobutyl](4aS,8aS)-isochinolin-3(S)-carboxamid in Form eines crèmefarbenen Festkörpers erhalten wurden, MS: m/e 532 [M+H]⁺.

Das als Ausgangsmaterial verwendete 2(S)-[2-Phenyl-1(S)-phthalimidoäthyl]oxiran wurde wie folgt hergestellt:
(i) Ein Gemisch von 134 g 3-Phenyl-2(S)-phthalimidopropionylchlorid und 269,7 g Tris(trimethylsilyloxy)äthylen wurde 5 Stunden bei 95°C gerührt. Weitere 54 g Tris(trimethylsilyloxy)äthylen wurden zugegeben, und das Reaktionsgemisch weitere 1,5 Stunden bei 95°C gerührt. Das abgekühlte Reaktionsgemisch wurde danach mit 435 ml Dioxan und 174 ml 0,6N Salzsäure versetzt, und das erhaltene Gemisch 30 Minuten auf 85°C erhitzt. Das abgekühlte Gemisch wurde mit Natriumchlorid gesättigt und danach mit Diäthyläther extrahiert. Die Aetherlösungen wurden mit wässriger Natriumbicarbonatlösung gewaschen und eingedampft. Der erhaltene leicht gelbliche Festkörper wurde aus Aethylacetat/n-Hexan kristallisiert, wobei 103 g N-[1(S)-Benzyl-3-hydroxy-2-oxopropyl]phthalimid als weisser Festkörper erhalten wurden, MS: m/e 310 [M+H]⁺.
(ii) 100 ml 4N Salzsäure in Aethylacetat wurden tropfenweise unter Rühren zu einer Suspension von 120,4 g N-[1(S)-Benzyl-3-hydroxy-2-oxopropyl]phthalimid in 355 ml Dihydropyran gegeben. Nach 20 Minuten wurde das Gemisch mit 500 ml Aethylacetat verdünnt, mit Natriumbicarbonatlösung und Natriumchloridlösung gewaschen und eingedampft. Das erhaltene braune Oel wurde in 3,9 l wässrigem Tetrahydrofuran (10%) gelöst, und die Lösung unter Zugabe von 7,36 g Natriumborhydrid bei -10°C bis -7°C gerührt. Nach 20-minütigem Rühren bei -7°C erwärmte man das Gemisch auf 0°C, gab weitere 7,36 g Natriumborhydrid zu und rührte das Gemisch weitere 5 Minuten bei 0°C. Das Reaktionsgemisch wurde danach mit 1 l Wasser verdünnt, und der pH-Wert durch Zugabe von konzentrierter Salzsäure auf 6 eingestellt. Das Tetrahydrofuran wurde durch Abdampfen entfernt, und die zurückbleibende wässrige Lösung mit Dichlormethan extrahiert. Die organische Lösung wurde mit Natriumchloridlösung gewaschen und eingedampft. Das erhaltene braune Oel wurde in 1,1 l Pyridin gelöst, und die Lösung unter Zugabe von 89 g Methansulfonylchlorid bei 20°C gerührt. Das Gemisch wurde danach weitere 50 Minuten gerührt und dann auf 2 kg zerkleinertes Eis gegossen. Das Gemisch wurde durch Zugabe von konzentrierter Schwefelsäure auf pH 2 eingestellt und dann mit Dichlormethan extrahiert. Die organische Lösung wurde mit Natriumchloridlösung gewaschen und eingedampft. Das erhaltene braune Oel wurde in 1 l Aethanol gelöst und mit 11 g p-Toluolsulfonsäure versetzt. Das Gemisch wurde 1 Stunde bei 20°C gerührt und danach weitere 20 Minuten bei 0°C. Der erhaltene Niederschlag wurde abfiltriert und mit Diäthyläther gewaschen, wobei 38,57 g N-[1(S)-Benzyl-3-hydroxy-2(R)-(methylsulfonyloxy)propyl]phthalimid als weisser Festkörper erhalten wurden, MS: m/e 389 [M]⁺.
(iii) 2,16 g N-[1(S)-Benzyl-3-hydroxy-2(R)-(methylsulfonyloxy)propyl]phthalimid wurden zu einer gerührten Suspension von 200 mg Natriumhydrid (80%ige Dispersion in Mineralöl) in 50 ml trockenem Tetrahydrofuran gegeben, und das Reaktionsgemisch 2 Stunden bei 20°C gerührt. Weitere 100 mg Natriumhyrid wurden zugegeben, und das Rühren für 3,5 Stunden fortgesetzt. Das Gemisch wurde mit Dichlormethan und Wasser verdünnt, und der pH-Wert der wäsrigen Phase durch Zugabe von 2M Salzsäure auf 7 eingestellt. Die organische Phase wurde abgetrennt, mit gesättigter Natriumchloridlösung gewaschen und eingedampft. Der Rückstand wurde unter Verwendung von Aethylacetat/n-Hexan (1:2) als Eluierungsmittel an Silicagel chromatographiert, wobei man 0,67 g 2(S)-[2-Phenyl-1(S)-phthalimidoäthyl]oxiran als weissen Festkörper erhielt, MS: m/e 294 [M+H]⁺.

Das als Ausgangsmaterial verwendete N-tert.Butyl-decahydro-(4aS,8aS)-isochinolin-3(S)-carboxamid wurde wie folgt hergestellt:
i) Eine Suspension von 12,676 g (71,6 mMol) 1,2,3,4-Tetrahydro-3(S)-isochinolincarbonsäure (Chem. Pharm. Bull. 1983, 31, 312) in 200 ml 90%iger Essigsäure wurde bei 80°C und unter einem Druck von 140 Atmosphären über 5% Rhodium auf Kohlenstoff 24 Stunden hydriert. Man liess danach das Reaktionsgemisch auf Raumtemperatur abkühlen und filtrierte den Katalysator ab. Das Filtrat wurde zu einem Gummi eingedampft, welcher in 10 ml Aethylacetat gelöst und unter starkem Rühren langsam zu 100 ml Diisopropyläther gegeben wurde. Dabei entstand ein harzartiger Niederschlag. Die überstehende Lösung wurde durch Dekantieren entfernt, und der Niederschlag mit heissem Aethylacetat extrahiert. Diese heisse Lösung wurde unter starkem Rühren zu einem Gemisch von 150 ml Diäthyläther/Diiso-propyläther (1:1) unter Bildung eines leicht grauen Festkörpers gegossen, welcher abfiltriert, mit Diäthyläther gewaschen und getrocknet wurde. Auf diese Weise wurden 5,209 g eines Gemisches von Decahydroisochinolin-3(S)-carbonsäuren erhalten, welches zur Hauptsache (ungefähr 65%) aus dem 4aS,8aS-Isomeren bestand, zusammen mit den 4aR,8aR-lsomeren (ungefähr 25%) und ungefähr 10% der trans-Isomeren, MS: m/e 184 [M+H]⁺.
(ii) 9,036 g (49,4 mMol) des oben genannten Gemisches der Decahydroisochinolin-3(S)-carbonsäuren wurden in 50 ml (50 mMol) 1M Natriumhydroxidlösung gelöst, und die erhaltene Lösung auf 0°C gekühlt. 7,40 ml (51,87 mMol) Benzylchloroformat und 58,7 ml (58,7 mMol) 1M Natriumhydroxidlösung wurden über einen Zeitpunkt von 1 Stunde tropfenweise zugegeben, wobei die Temperatur durch Kühlen auf 0-5°C gehalten wurde. Das Gemisch wurde dann 2 Stunden weitergerührt, wobei man während dieser Zeit das Gemisch auf Raumtemperatur aufwärmen liess. 100 ml Diäthyläther wurden zugegeben, und das Gemisch filtriert, wobei das unlösliche R,R-Isomere entfernt wurde. Die wässrige Phase des Filtrats wurde abgetrennt und durch Zugabe von konzentrierter Salzsäure auf pH 1,5-2 eingestellt, wobei sich ein Oel abschied. Das Gemisch wurde zweimal mit je 100 ml Aethylacetat extrahiert. Die vereinigten organischen Extrakte wurden mit Wasser gewaschen, über wasserfreiem Natriumsulfat getrocknet und zu einem Oel eingedampft. Dieses Oel wurde in 35 ml Aethylacetat gelöst, und 2,85 ml (25 mMol) Cyclohexylamin wurden dazugegeben. Der weisse Niederschlag wurde abfiltriert und lieferte nach einigen fraktionierten Umkristallisationen aus Methanol/Aethylacetat 2,38g g des Cydohexylaminsalzes von 2-(Benzyloxycarbonyl)-decahydro-(4aS,8aS)-isochinolin-3(S)-carbonsäure, MS: m/e 318 [M+H]⁺.
(iii) 2,384 g des Cydohexylaminsalzes von 2-(Benzyloxycarbonyl)-decahydro-(4aS,8aS)-isochinolin-3(S)-carbonsäure wurden zwischen 50 ml Aethylacetat und 50 ml 10%iger Zitronensäurelösung verteilt. Die organische Phase wurde abgetrennt, mit Wasser gewaschen, filtriert und eingedampft, wobei 1,87 g 2-(Benzyloxycarbonyl)-decahydro-(4aS,8aS)-isochinolin-3(S)-carbonsäure in Form eines farblosen Gummis erhalten wurden, MS: m/e 318 [M+H]⁺.
(iv) Eine Lösung von 0,634 g (2,0 mMol) 2-(Benzyloxycarbonyl)-decahydro-(4aS,8aS)-isochinolin-3(S)-carbonsäure in 6 ml Dimethoxyäthan wurde mit 0,23 g (2,0 mMol) N-Hydroxysuccinimid und 0,412 g (2,0 mMol) Dicyclohexylcarbodiimid versetzt. Das Gemisch wurde 18 Stunden bei Raumtemperatur gerührt. Danach wurde das Gemisch abfiltriert, und das Filtrat eingedampft, wobei 0,879 g des N-Hydroxysuccinimidesters der oben genannten Säure in Form eines leicht gelblichen Oel erhalten wurde. Eine Lösung von 0,828 g (2,0 mMol) des obigen N-Hydroxysuccinimidesters in 5 ml Dichlormethan wurde gerührt, auf 0°C abgekühlt und mit 0,219 g (3,0 mMol) tert.Butylamin versetzt. Das Gemisch wurde zunächst 2 Stunden bei 0°C und danach 4,5 Stunden bei Raumtemperatur gerührt. Das Gemisch wurde dann nacheinander mit 2M Salzsäure, Natriumcarbonatlösung und Natriumchloridlösung gewaschen, über wasserfreiem Magnesiumsulfat getrocknet und eingedampft. Der Rückstand wurde in 20 ml Diäthyläther gelöst und filtriert. Das Filtrat wurde eingedampft, wobei man 0,712 g 2-(Benzyloxycarbonyl)-N-tert.butyl-decahydro-(4aS,8aS)-isochinolin-3(S)-carboxamid in Form eines leicht gelblichen Glases erhielt, MS: m/e 373 [M+H].
(v) Eine Lösung von 0,689 g (1,85 mMol) 2-(Benzyloxycarbonyl)-N-tert.butyl-decahydro-(4aS,8aS)-isochinolin-3(S)-carboxamid in 20 ml Aethanol wurde in Gegenwart von 0,01 g 10%igem Palladium auf Kohle bei Raumtemperatur und Atmosphärendruck während 18 Stunden hydriert. Der Katalysator wurde abfiltriert, und das Lösungsmittel abgedampft, wobei 1,85 mMol N-tert.Butyl-decahydro-(4aS,8aS)-isochinolin3(S)-carboxamid als klares Oel erhalten wurden, MS: m/e 239 [M+H]⁺, welches ohne weitere Reinigung in die nächste Stufe eingesetzt wurde.

### Beispiel 2

Ein Gemisch von 0,378 g (2 mMol) 2(S)-[1(S)-Azido-2-phenyläthyl]oxiran und 0,476 g (2 mMol) N-tert.Butyl-decahydro-(4aS,8aS)-isochinolin-3(S)-carboxamid in 10 ml Aethanol wurde unter Rühren 5 Stunden zum Rückfluss erhitzt und danach 18 Stunden bei Raumtemperatur stehengelassen. Das Gemisch wurde unter vermindertem Druck zur Trockene eingedampft, und der erhaltene leicht gelbe Festkörper aus Aethylacetat/n-Hexan (1:1) kristallisiert, wobei man 0,593 g eines weissen Festkörpers erhielt. Umkristallisation aus dem gleichen Lösungsmittelgemisch lieferte 0,165 g 2-[3(S)-Azido-2(R)-hydroxy-4-phenylbutyl]-N-tert.butyl-decahydro-(4aS,8aS)-isochinolin-3(S)-carboxamid, MS: m/e 428 [M+H]⁺.

Das als Ausgangsmaterial verwendete 2(S)-[1(S)-Azido-2-phenyläthyl]oxiran wurde wie folgt hergestellt:
(i) 4,34 g (0,02 Mol) (2R,3R)-2-Benzyloxybutan-1,3,4-triol wurden in einem Gemisch von 3,4 g (0,02 Mol) 1-Cyclohexenyloxytrimethylsilan und 40 ml Diäthyläther suspendiert. 2 Tropfen konzentrierte Salzsäure wurden zugegeben, und das Gemisch 30 Minuten bei Raumtemperatur gerührt. Das Gemisch wurde dann eingedampft, wobei 5,65 g β(R)-Benzyloxy-1,4-dioxaspiro[4,5]decan-2(R)-äthanol in Form eines farblosen viskosen Oels erhalten wurden, MS: m/e 292 [M]⁺.
(ii) 15,0 g Pyridiniumchlorchromat und 10,0 g zerkleinertes 3A Molekularsieb wurden zu einer Lösung von 5,50 g (0,019 Mol) β(R)-Benzyloxy-1,4-dioxaspiro[4,5]-decan-2(R)-äthanol in 200 ml Dichlormethan gegeben. Das Gemisch wurde 20 Stunden bei Raumtemperatur gerührt, dann mit 200 ml Diäthyläther verdünnt und filtriert. Das Filtrat wurde eingedampft, wobei 4,30 g α(S)-Benzyloxy-1,4-dioxaspiro[4,5]decan-2(R)-acetaldehyd erhalten wurden, welche ohne weitere Reinigung in die nächste Stufe eingesetzt wurden.
(iii) Eine Lösung von 4,30 g (0,015 Mol) α(S)-Benzyloxy-1,4-dioxaspiro[4,5]decan-2(R)-acetaldehyd in 50 ml wasserfreiem Diäthyläther wurde auf -8°C gekühlt und innerhalb 1 Stunde tropfenweise mit 15 ml einer 2M Lösung von Phenylmagnesiumchlorid in Tetrahydrofuran versetzt. Man rührte das Reaktionsgemisch 2 Stunden bei -5°C, liess es danach auf Raumtemperatur erwärmen und goss es in 50 ml einer 10%igen Ammoniumchloridlösung. Das Gemisch wurde zweimal mit je 100 ml Aethylacetat extrahiert. Die Aethylacetatextrakte wurden über wasserfreiem Magnesiumsulfat getrocknet und eingedampft. Der Rückstand wurde unter Verwendung von 5% Methanol in Dichlormethan als Eluierungsmittel an Silicagel flash-chromatographiert. Auf diese Weise wurden 2,47 g β(R)-Benzyloxy-α(RS)-phenyl-1,4-dioxaspiro[4,5]decan-2(R)-äthanol in Form eines farblosen Gummis erhalten, MS (C.I.): m/e 369 [M+H]⁺.
(iv)(a) Eine Lösung von 130 mg (0,35 mMol) β(R)-Benzyloxy-α(RS)-phenyl-1,4-dioxaspiro[4,5]decan-2(R)-äthanol in 3 ml Essigsäure wurde über 10% Palladium auf Kohle unter einem Druck von ungefähr 4,1 Atmosphären 2 Tage hydriert. Der Katalysator wurde abfiltriert, und das Filtrat eingedampft. Das Rohprodukt wurde durch Flash-Chromatographie an Silicagel unter Verwendung von 2% Methanol in Dichlormethan als Eluierungsmittel gereinigt. So wurden 60 mg α(R)-Benzyl-1,4-dioxaspiro[4,5]decan-2(R)-methanol in Form eines farblosen Oels erhalten, MS: m/e 262 [M]⁺..
(iv)(b) Eine Lösung von 80 mg (0,22 mMol) β(R)-Benzyloxy-α(RS)-phenyl-1,4-dioxaspiro[4,5]-2(R)-äthanol und 50 mg (0,49 mMol) Essigsäureanhydrid in 1 ml Pyridin wurde 20 Stunden bei Raumtemperatur gerührt. Die Lösungsmittel wurden durch Abdampfen entfernt, wobei 80 mg β(R)-Benzyloxy-α(RS)-phenyl-1,4-dioxaspiro[4,5]decan-2(R)-äthylacetat als farbloses Oel erhalten wurden, MS: m/e 410 [M]⁺.

Eine Lösung von 80 mg β(R)-Benzyloxy-α(RS)-phenyl-1,4-dioxaspiro[4,5]-decan-2(R)-äthylacetat in 3 ml Essigsäure wurde über 10% Palladium auf Kohle unter einem Druck von ungefähr 4,8 Atmosphären 2 Tage hydriert. Der Katalysator wurde abfiltriert, und das Filtrat eingedampft. Das Rohprodukt wurde an Silicagel unter Verwendung von 2% Methanol in Dichlormethan als Eluierungsmittel flash-chromatographiert. So wurden 33 mg α(R)-Benzyl-1,4-dioxaspiro[4,5]decan-2(R)-methanol in Form eines farblosen Oels erhalten, MS: m/e 262 [M]⁺.
(v) Ein Gemisch von 65 mg (0,25 mMol) α(R)-Benzyl-1,4-dioxaspiro[4,5]-decan-2(R)-methanol, 65 mg (0,28 mMol) Triphenylphosphin und 80 mg (1,2 mMol) Natriumazid in 1 ml Dimethylformamid wurde auf 0°C gekühlt und mit 83 mg (0,25 mMol) Kohlenstofftetrabromid versetzt. Das Gemisch wurde bei Raumtemperatur über Nacht gerührt. 0,5 ml Methanol wurde zugegeben, und das Gemisch 15 Minuten gerührt und danach zur Trockene eingedampft. Der Rückstand wurde zwischen 10 ml Aethylacetat und 10 ml Wasser verteilt, die organische Phase abgetrennt, über wasserfreiem Magnesiumsulfat getrocknet und eingedampft. Der Rückstand wurde durch Flash-Chromatographie an Silicagel unter Verwendung von 20% Aethylacetat in n-Hexan als Eluierungsmittel gereinigt. So wurden 48 mg 2(S)-[1(S)-Azido-2-phenyläthyl]-1,4-dioxaspiro[4,5]decan als farbloses Oel erhalten, MS (C.I.): m/e 260 [M+H-N₂]⁺.
(vi) Eine Lösung von 138 mg (0,48 mMol) 2(S)-[1(S)-Azido-2-phenyläthyl]-1,4-dioxaspiro[4,5]decan in einem Gemisch von 8 ml Essigsäure und 2 ml Wasser wurde bei 100°C 2 Stunden gerührt. Das Lösungsmittel wurde durch Abdampfen entfernt, und der Rückstand zwischen 15 ml Aethylacetat und 10 ml gesättigter Natriumbicarbonatlösung verteilt. Die organische Phase wurde über wasserfreiem Magnesiumsulfat getrocknet und eingedampft, wobei 85 mg eines weissen Festkörpers erhalten wurden. Dieser wurde in 5 ml 10% Aethylacetat in n-Hexan suspendiert, 15 Minuten gerührt und abfiltriert, wobei 34 mg 3(S)-Azido-4-phenyl-1,2(S)-butandiol in Form eines weissen Festkörpers erhalten wurden, Schmelzpunkt 83-84°C.
(vii) 1,05 g (5,5 mMol) p-Toluolsulfonylchlorid wurden zu einer Lösung von 0,95 g (4,6 mMol) 3(S)-Azido-4-phenyl-1,2(S)-butandiol und 30 mg 4-Dimethylaminopyridin in einem Gemisch von 30 ml Dichlormethan und 5 ml Pyridin gegeben. Die erhaltene Lösung wurde 48 Stunden bei Raumtemperatur gerührt. Das Lösungsmittel wurde unter vermindertem Druck entfernt, und der ölige Rückstand zwischen Wasser und Aethylacetat verteilt. Die wässrige Phase wurde zweimal mit Aethylacetat rückextrahiert. Die organischen Phasen wurden vereinigt, mit 10%iger wässriger Schwefelsäure und Natriumchloridlösung gewaschen, über wasserfreiem Magnesiumsulfat getrocknet und zur Trockene eingedampft. Reinigung des Rohprodukts durch Flash-Chromatographie an Silicagel unter Verwendung von n-Hexan/Aethylacetat (3:1) als Eluierungsmittel lieferte 1,37 g 3(S)-Azido-4-phenyl-1-(p-toluolsulfonyloxy)-2(S)-butanol in Form eines viskosen Oel, MS: m/e 362 [M+H]⁺.
(viii) Eine Lösung von 280 mg (5 mMol) Kaliumhydroxid in 10 ml Aethanol wurde zu einer Lösung von 1,37 g (3,8 mMol) 3(S)-Azido-4-phenyl-1-(p-toluolsulfonyloxy)-2(S)-butanol in 50 ml Aethanol gegeben, und das Gemisch 1 Stunde bei Raumtemperatur gerührt. Das Gemisch wurde danach zur Trockene eingedampft, und der Rückstand zwischen Wasser und Dichlormethan verteilt. Die wässrige Phase wurde zweimal mit Dichlormethan rückextrahiert. Die organischen Phasen wurden vereinigt, über wasserfreiem Magnesiumsulfat getrocknet und eingedampft, wobei ein Oel erhalten wurde, welches durch Flash-Chromatographie an Silicagel unter Verwendung von n-Hexan/Aethylacetat (4:1) als Eluierungsmittel gereinigt wurde. So erhielt man 0,47 g 2(S)-[1(S)-Azido-2-phenyläthyl]oxiran, MS: m/e 189 [M]⁺.

### Beispiel 3

Eine Lösung von 237 mg (1 mMol) N-tert.Butyl-decahydro-(4aS,8aS)-isochinolin-3(S)-carboxamid und 300 mg (1,1 mMol) 4(S)-[1(S)-Azido-2-phenyläthyl]-1,3,2-dioxathiolan-2,2-dioxid in 10 ml Tetrahydrofuran wurde unter einer Argonatmosphäre 2 Tage bei Raumtemperatur gerührt, wobei sich während dieser Zeit etwas weisser Niederschlag bildete. Das Gemisch wurde zu einem weissen Puder eingedampft, welcher in einer 10%igen Lösung von Schwefelsäure in 70%igem wässrigem Methanol aufgenommen und 30 Minuten zum Rückfluss erhitzt wurde. Nach Abkühlen auf Raumtemperatur wurde die Lösung mit 10%iger wässriger Natriumhydroxidlösung auf pH 10 eingestellt und dreimal mit Aethylacetat extrhahiert. Die Aethylacetatextrakte wurden vereinigt, mit Natriumchloridlösung gewaschen, über wasserfreiem Magnesiumsulfat getrocknet und eingedampft. Reinigung des Rohprodukts durch Flash-Chromatographie an Silicagel mit n-Hexan/Aethylacetat (4:1) als Eluierungsmittel lieferte 57 mg 2-[3(S)-Azido-2(R)-hydroxy-4-phenylbutyl]-N-tert.butyl-decahydro-(4aS,8aS)-isochinolin-3(S)-carboxamid, MS: m/e 428 [M+H]⁺.

Das als Ausgangsmaterial verwendete 4(S)-[1(S)-Azido-2-phenyläthyl]-1,3,2-dioxathiolan-2,2-dioxid wurde wie folgt hergestellt:

175 µl (2,4 mMol) Thionylchlorid wurden zu einer Lösung von 414 mg (2 mMol) 3(S)-Azido-4-phenyl-1,2(S)-butandiol [welches wie in Beispiel 2(vi) hergestellt wurde] in 5 ml Tetrachlorkohlenstoff gegeben, und das durch ein Calciumchlorid-Trocknungsrohr geschützte Gemisch wurde 30 Minuten zum Rückfluss erhitzt. Die erhaltene Lösung wurde in einem Eisbad gekühlt, nacheinander mit 5 ml Acetonitril, 5 mg Ruthenium(III)chlorid-trihydrat, 642 mg (3 mMol) Natriummetaperjodat und 7,5 ml Wasser versetzt, und schliesslich während 1 Stunde bei Raumtemperatur intensiv gerührt. Das Gemisch wurde mit 20 ml Diäthyläther und 20 ml Wasser versetzt, die Phasen wurden getrennt, und die wässrige Phase wurde zweimal mit Diäthyläther extrahiert. Die organischen Phasen wurden vereinigt und mit Wasser, gesättigter wässriger Natriumbicarbonatlösung und Natriumchloridlösung gewaschen. Nach dem Trocknen über wasserfreiem Magnesiumsulfat, Filtrieren durch Diatomeenerde und Eindampfen des Filtrates wurden 518 mg 4(S)-[1(S)-Azido-2-phenyläthyl]-1,3,2-dioxathiolan-2,2-dioxid erhalten, MS: m/e 270 [M+H]⁺.

Die folgenden Beispiele veranschaulichen die Ueberführung von Alkoholen der Formel I in Aminosäurederivate der Formel II:

### Beispiel 4

(A)(i) Ein Gemisch von 25,6 g (48,3 mMol) N-tert.Butyl-decahydro-2-[2(R)-hydroxy-4-phenyl-3(S)-phthalimidobutyl]-(4aS,8aS)-isochinolin-3(S)-carboxamid (welches wie in Beispiel 1 beschrieben hergestellt wurde) und 3,74 ml (96,5 mMol) Hydrazinhydrat in 145 ml Aethanol wurde 4 Stunden bei Raumtemperatur gerührt. Das Gemisch wurde unter vermindertem Druck zur Trockene eingedampft. Der Rückstand wurde in Toluol gelöst, und die Lösung eingedampft; dieses Prozedere wurde einmal wiederholt. Der Rückstand wurde dann in 2M Essigsäure gelöst, und die Lösung 1 Stunde bei Raumtemperatur gerührt. Das Gemisch wurde filtriert, und das Filtrat durch Zugabe von festem Natriumcarbonat basisch gestellt und danach zweimal mit Dichlormethan extrahiert. Die vereinigten Dichlormethanextrakte wurden mit Natriumchloridlösung gewaschen, filtriert und eingedampft, wobei ein brauner Festkörper erhalten wurde, welcher mit Diäthyläther angerieben wurde. So wurden 12,93 g 2-[3(S)-Amino-2(R)-hydroxy-4-phenylbutyl]-N-tert.butyl-decahydro-(4aS,8aS)-isochinolin-3(S)-carboxamid als weisser Festkörper erhalten, MS: m/e 402 [M+H]⁺.
(A)(ii) Eine Suspension von 26,55 g N-tert.Butyl-2-[2(R)-hydroxy-4-phenyl-3(S)-phthalimidobutyl]-(4aS,8aS)-isochinolin-3(S)-carboxamid (welches wie in Beispiel 1 beschrieben hergestellt wurde) in 100 ml Aethanol wurde mit 25 ml einer 30%igen Lösung von Methylamin in Aethanol 1 Stunde bei Raumtemperatur behandelt. Das Gemisch wurde unter vermindertem Druck zur Trockene eingedampft. Der Rückstand wurde in 250 ml Aethanol gelöst, mit 250 ml einer gesättigten Lösung von Chlorwasserstoff in Aethylacetat versetzt und 16 Stunden bei Raumtemperatur gerührt. Das Gemisch wurde zur Trockene eingedampft, und der Rückstand zwischen Aethylacetat und Wasser verteilt. Die wässrige Phase wurde abgetrennt, mit festem Natriumcarbonat basisch gestellt und dann mit Dichlormethan extrahiert. Der Dichlormethanextrakt wurde mit Natriumchloridlösung gewaschen und zur Trockene eingedampft. Der Rückstand wurde mit Diäthyläther angerieben, und nach Filtrieren wurden so 17,48 g 2-[3(S)-Amino-2(R)-hydroxy-4-phenylbutyl]-N-tert.butyl-decahydro-(4aS,8aS)-iscchinolin-3(S)-carboxamid als weisser Festkörper erhalten, MS: m/e 402 [M+H]⁺.
(A)(iii) Eine Lösung von 17 mg (0,04 mMol) 2-[3(S)-Azido-2(R)-hydroxy-4-phenylbutyl]-N-tert.butyl-decahydro-(4aS,8aS)-isochinolin-3(S)-carboxamid (welches wie in Beispiel 2 oder Beispiel3 beschrieben hergestellt wurde) in 5 ml Aethanol wurde über 10 mg 10%igem Palladium auf Kohle 2 Stunden bei Raumtemperatur und ungefähr 3,4 Atmosphären Druck hydriert. Der Katalysator wurde abfiltriert und zweimal mit Aethanol gewaschen. Die vereinigten Filtrate und Waschlösungen wurden eingedampft, wobei 16 mg 2-[3(S)-Amino-2(R)-hydroxy-4-phenylbutyl]-N-tert.butyl-decahydro-(4aS,8aS)-isochinolin-3(S)-carboxamid erhalten wurden, MS: m/e 402 [M+H]⁺.
(B) Eine Lösung von 561 mg 2-[3(S)-Amino-2(R)-hydroxy-4-phenylbutyl]-N-tert.butyl-decahydro-(4aS,8aS)-isochinolin-3(S)-carboxamid und 372 mg N-(Benzyloxycarbonyl)-L-asparagin in 20 ml trockenem Tetrahydrofuran wurde in einem Eis/Salz-Gemisch gekühlt. 189 mg Hydroxybenzotriazol, 161 mg N-Aethylmorpholin und 317 mg Dicyclohexylcarbodiimid wurden zugegeben, und das Gemisch 16 Stunden gerührt. Das Gemisch wurde danach mit Aethylacetat verdünnt und filtriert. Das Filtrat wurde mit wässriger Natriumbicarbonatlösung und Natriumchloridlösung gewaschen. Das Lösungsmittel wurde durch Abdampfen entfernt, und der Rückstand an Silicagel mit Dichlormethan/Methanol (9:1) als Eluierungsmittel chromatographiert, wobei 434 mg 2-[3(S)-[[N-(Benzyloxycarbonyl)-L-asparaginyl]amino]-2(R)-hydroxy-4-phenylbutyl]-N-tert.butyl-decahydro-(4aS,8aS)-isochinolin-3(S)-carboxamid in Form eines weissen Festkörpers aus Methanol/Diäthyläther erhalten wurden, MS: m/e 650 (M+H)⁺.

### Beispiel 5

Eine Lösung von 287 mg N-(2-Chinolylcarbonyl)-L-asparagin und 401 mg 2-[3(S)-Amino-2(R)-hydroxy-4-phenylbutyl]-N-tert.butyl-decahydro-(4aS,8aS)-isochinolin-3(S)-carboxamid (welches wie in Beispiel 5 beschrieben hergestellt wurde) in 3 ml Tetrahydrofuran wurde auf -10°C abgekühlt und mit 163 mg 3-Hydroxy-1,2,3-benzotriazin-4(3H)-on und 220 mg Dicydohexylcarbodiimid versetzt. Das Gemisch wurde 2 Stunden bei -10°C und 16 Stunden bei 20°C gerührt, dann mit Aethylacetat verdünnt und filtriert. Das Filtrat wurde mit gesättigter Natriumbicarbonatlösung und gesättigter Natriumchloridlösung gewaschen und eingedampft. Der Rückstand wurde unter Verwendung von 4 Volumenprozent Methanol in Dichlormethan als Eluierungsmittel an Silicagel chromatographiert, wobei 537 mg N-tert.Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid als weisser Festkörper erhalten wurden, MS: m/e 671 (M+H)⁺.

Das als Ausgangsmaterial verwendete N-(2-Chinolylcarbonyl)-L-asparagin wurde wie folgt hergestellt:

Ein Gemisch von 540 mg Chinalidinsäuresuccinimidester und 300 mg L-Asparaginmonohydrat in 2 ml Dimethylformamid wurde 96 Stunden bei 20°C gerührt. Das Lösungsmittel wurde durch Abdampfen entfernt, wobei ein weisser Festkörper erhalten wurde. Dieser Festkörper wurde in 10 ml Dichlormethan kräftig gerührt, abfiltriert und mit Dichlormethan gewaschen. Auf diese Weise wurden 431 mg N-(2-Chinolylcarbonyl)-L-asparagin als weisser Festkörper erhalten, MS: m/e 288 (M+H)⁺.

## Patentansprüche

1. Alkohole der allgemeinen Formel 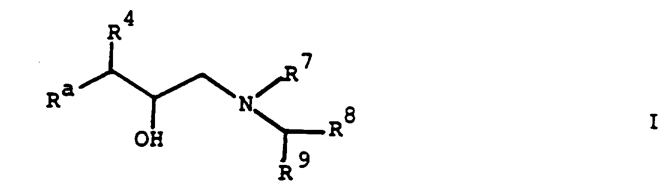 worin R^{a} Azido oder Phthaliimido, R⁴ Alkyl, Cycloalkyl, Cycloalkylalkyl, Aryl oder Aralkyl und R⁷ und R⁸ zusammen eine Trimethylen-oder Tetramethylengruppe bedeuten, welche gegebenenfalls durch Hydroxy, Alkoxycarbonylamino oder Acylamino substituiert ist oder in welcher eine -CH₂-Gruppe durch -NH-, -N(Alkoxycarbonyl)-, -N(Acyl)-oder -S- ersetzt ist oder welche einen anellierten Cycloalkan-, aromatischen oder heteroaromatischen Ring trägt, und R⁹ Alkoxycarbonyl, Monoalkylcarbamoyl, Monoaralkylcarbamoyl, Monoarylcarbamoyl oder eine Gruppe der Formel 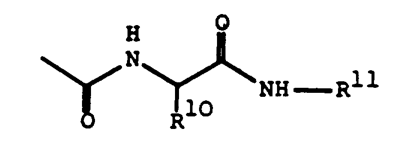 bedeutet, in welcher R¹⁰ und R¹¹ je Alkyl bedeuten.

2. Alkohole gemäss Anspruch 1, worin R^{a} Phthalimido bedeutet.

3. Alkohole gemäss Anspruch 1 oder 2, worin R⁴ Aralkyl bedeutet.

4. Alkohole gemäss einem der Ansprüche 1-3, worin R⁴ Benzyl bedeutet.

5. Alkohole gemäss einem der Ansprüche 1-4, worin -N(R⁷)-CH(R⁸)(R⁹) eine Gruppe der Formel 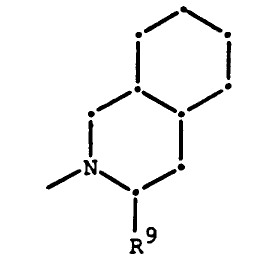 bedeutet, in welcher R⁹ Monoalkylcarbamoyl bedeutet.

6. Alkohole gemäss Anspruch 5, worin R⁹ tert.Butylcarbamoyl bedeutet.

7. Alkohole gemäss einem der Ansprüche 1-5, worin R^{a} Phthalimido, R⁴ Benzyl und -N(R⁷)-CH(R⁸)(R⁹) eine Gruppe der in Anspruch 5 angegebenen Formel bedeuten, in welcher R⁹ tert.Butylcarbamoyl bedeutet.

8. N-tert.Butyl-decahydro-2-[2(R)-hydroxy-4-phenyl-3(S)-phthalimidobutyl]-(4aS,8aS)-isochinolin-3(S)-carboxamid.

9. 2-[3(S)-Azido-2(R)-hydroxy-4-phenylbutyl]-N-tert.butyl-decahydro-(4aS,8aS)-isochinolin-3(S)-carboxamid.

10. Epoxide der allgemeinen Formel 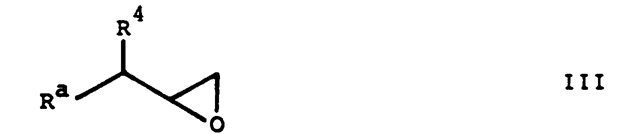 worin R^{a} Azido oder Phthalimido, und R⁴ Alkyl, Cycloalkyl, Cycloalkylalkyl, Aryl oder Aralkyl bedeuten, wobei falls R⁴ Hexyl ist, R^{a} nicht Azido und falls R⁴ Isobutyl, R^{a} nicht Phthalimido sein soll.

11. 2(S)-[2-Phenyl-1(S)-phthalimidoäthyl]oxiran.

12. 2(S)-[1(S)-Azido-2-phenyläthyl]oxiran.

13. Verbindungen der allgemeinen Formel 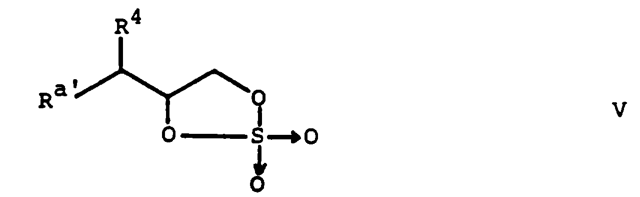 worin R^{a'} Azido und R⁴ Alkyl, Cycloalkyl, Cycloalkylalkyl, Aryl oder Aralkyl bedeuten.

14. 4(S)-[1(S)-Azido-2-phenyläthyl]-1,3,2-dioxothiolan-2,2-dioxid.

15. Verfahren zur Herstellung von Alkoholen gemäss einem der Ansprüche 1-9, dadurch gekennzeichnet, dass man
(a) ein Epoxid der allgemeinen Formel 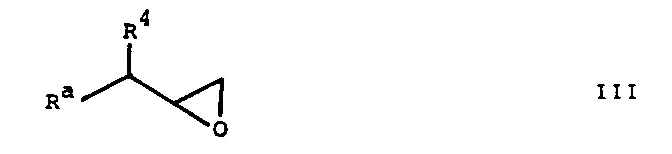 worin R^{a} und R⁴ die in Anspruch 1 angegebene Bedeutung besitzen, mit einer Verbindung der allgemeinen Formel 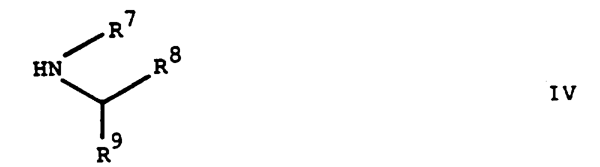 worin R⁷, R⁸ und R⁹ die in Anspruch 1 angegebene Bedeutung besitzen, umsetzt, oder
(b) zur Herstellung eines Alkohols der Formel I, worin R^{a} Azido bedeutet, eine Verbindung der allgemeinen Formel 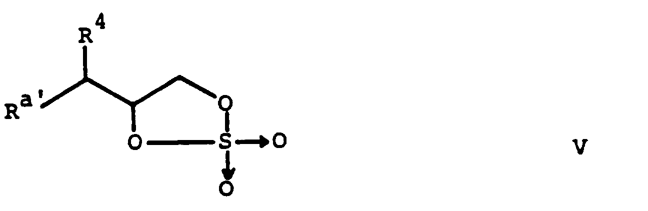 worin R^{a}' Azido bedeutet und R⁴ die in Anspruch 1 angegebene Bedeutung besitzt,
mit einer Verbindung der obigen Formel IV umsetzt.

16. Verwendung der Alkohole gemäss einem der Ansprüche 1-9 zur Herstellung von Aminosäurederivaten der allgemeinen Formel 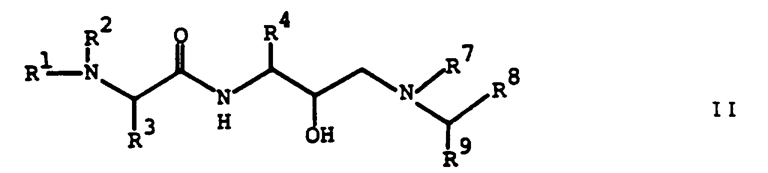 worin R¹ Alkoxycarbonyl, Aralkoxycarbonyl, Alkanoyl, Cycloalkylcarbonyl, Aralkanoyl, Aroyl, Heterocyclylcarbonyl, Alkylsulfonyl, Arylsulfonyl, Monoaralkylcarbamoyl, Cinnamoyl oder α-Aralkoxycarbonylaminoalkanoyl und R Wasserstoff oder R¹ und R zusammen mit dem Stickstoffatom, an welches sie gebunden sind, eine cyclische Imidgruppe der Formel 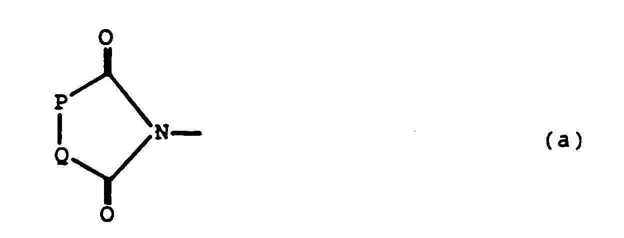 in welcher P und Q zusammen ein aromatisches System bedeuten, und R³ Alkyl, Cycloalkyl, Aryl, Aralkyl, Heterocyclylalkyl, Cyanoalkyl, Alkylthloalkyl, Alkylsulfinylalkyl, Carbamoylalkyl oder Alkoxycarbonylalkyl bedeuten und R⁴, R⁷, R⁸ und R⁹ die in Anspruch 1 angegebene Bedeutung besitzen.

17. Verwendung gemäss Anspruch 16 von N-tert.Butyl-decahydro-2-[2(R)-hydroxy-4-phenyl-3(S)-phthalimidobutyl]-(4aS,8aS)-isochinolin-3(S)-carboxamid zur Herstellung von N-tert.Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid.

## Claims

1. Alcohols of the general formula 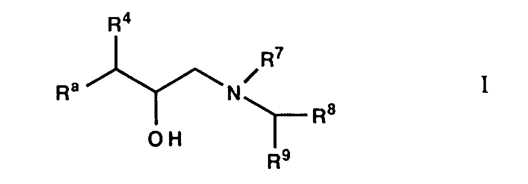 wherein R^{a} represents azido or phthalimido, R⁴ represents alkyl, cycloalkyl, cycloalkylalkyl, aryl or aralkyl and R⁷ and R⁸ rogether represent a trimethylene or tetramethylane group which is optionally substituted by hydroxy, alkoxycarbonylamino or acylamino or in which one -CH₂- group is replaced by -NH-, -N(alkoxycarbonyl)-, -N(acyl)- or -S- or which carried a fused cycloalkane, aromatic or heteroaromatic ring and R⁹ represents alkoxycarbonyl, monoalkylcarbamoyl, monoaralkylcarbamoyl, monoarylcarbamoyl or a group of the formula 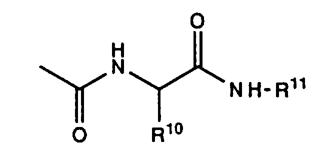 in which R¹⁰ and R¹¹ each represent alkyl.

2. Alcohols according to claim 1, wherein R^{a} represents phthalimido.

3. Alcohols according to claim 1 or 2, wherein R⁴ represents aralkyl.

4. Alcohols according to any one of claims 1-3, wherein R⁴ represents benzyl.

5. Alcohols according to any one of claims 1 to 4, wherein -N(R⁷)-CH(R⁸)(R⁹) represents a group of the formula 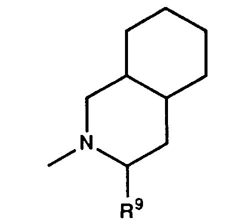 in which R⁹ represents monoalkylcarbamoyl.

6. Alcohols according to claim 5, wherein R⁹ represents tert.butylcarbamoyl.

7. Alcohols according to any one of claims 1-5, wherein R^{a} represents phthalimido, R⁴ represents benzyl and -N(R⁷)-CH(R⁸)(R⁹) represents a group of the formula given in claim 5 in which R⁹ represents tert.butylcarbamoyl.

8. N-tert.Butyl-decahydro-2-[2(R)-hydroxy-4-phenyl-3(S)-phthalimidobutyl]-(4aS,8aS)-isoquinoline-3(S)-carboxamide.

9. 2-[3(S)-Azido-2(R)-hydroxy-4-phenylbutyl]-N-tert.butyl-decahydro-(4aS,8aS)-isoquinoline-3(S)-carboxamide.

10. Epoxides of the general formula 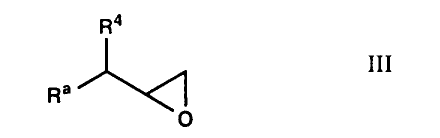 wherein R^{a} represents azido or phthalimido and R⁴ represents alkyl, cycloalkyl, cycloalkylalkyl, aryl or aralkyl, with the proviso that R^{a} is not azido when R⁴ is hexyl and R^{a} is not phthalimido when R⁴ is isobutyl.

11. 2(S)-[2-Phenyl-1(S)-phthalimidoethyl]oxirane.

12. 2(S)-[1(S)-Azido-2-phenylethyl]oxirane.

13. Compounds of the general formula 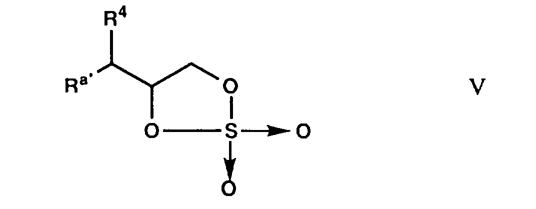 wherein R^{a'} represents azido and R⁴ represents alkyl, cycloalkyl, cycloalkylalkyl, aryl or aralkyl.

14. 4(S)-[1(S)-Azido-2-phenylethyl]-1,3,2-dioxothiolane 2,2-dioxide.

15. A process for the manufacture of alcohols according to any one of claims 1-9, characterized by
(a) reacting an epoxide of the general formula 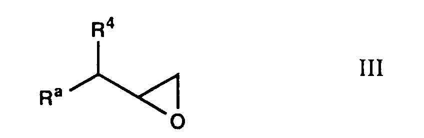 wherein R^{a} and R⁴ have the significance given in claim 1, with a compound of the general formula 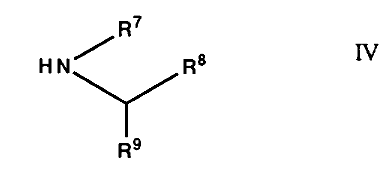 wherein R⁷, R⁸ and R⁹ have the significance given in claim 1,
or
(b) for the manufacture of an alcohol of formula I in which R^{a} represents azido, reacting a compound of the general formula 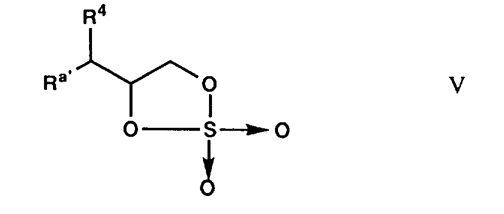 wherein R^{a'} represents azido and R⁴ has the significance given above,
with a compound of formula IV above.

16. The use of the alcohols according to any one of claims 1-9 for the manufacture of amino acid derivatives of the general formula 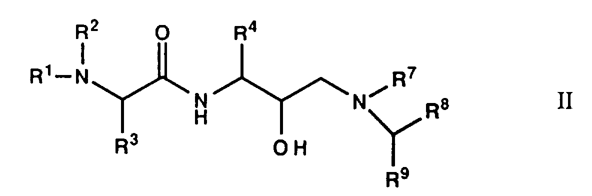 wherein R¹ represents alkoxycarbonyl, aralkoxycarbonyl, alkanoyl, cycloalkylcarbonyl, aralkanoyl, aroyl, heterocyclylcarbonyl, alkylsulphonyl, arylsulphonyl, monoaralkylcarbamoyl, cinnamoyl or α-aralkoxycarbonylaminoalkanoyl and R represents hydrogen or R¹ and R together with the nitrogen atom to which they are attached represent a cyclic imide group of the formula 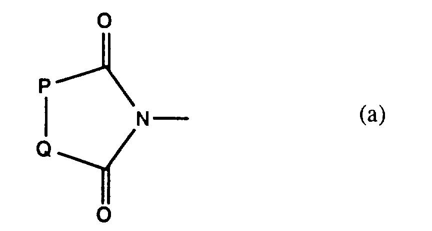 in which P and Q together represent an aromatic system, and R³ represents alkyl, cycloalkyl, aryl, aralkyl, heterocyclylalkyl, cyanoalkyl, alkylthioalkyl, alkylsulphinylalkyl, carbamoylalkyl or alkoxycarbonylalkyl and R⁴, R⁷, R⁸ and R⁹ have the significance given in claim 1.

17. The use according to claim 16 of N-tert.butyldecahydro-2-[2(R)-hydroxy-4-phenyl-3(S)-phthalimidobutyl]-(4aS,8aS)-isoquinoline-3(S)-carboxamide for the manufacture of N-tert.butyl-decahydro-2-[2(R)-hydroxy-4-phenyl-3(S)-[[N-(2-quinolylcarbonyl)-L-asparaginyl]amino]butyl]-(4aS,8aS)-isoquinoline-3(S)-carboxamide.

## Revendications

1. Alcools de formule générale 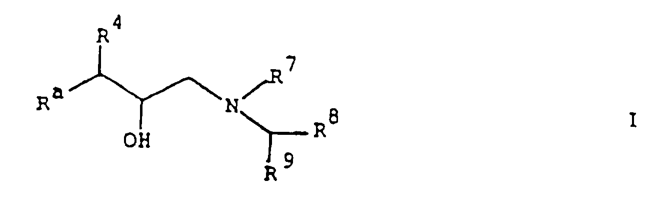 dans laquelle R^{a} représente le groupe azido ou phtalimido, R⁴ représente un groupe alkyle, cycloalkyle, cycloalkylalkyle, aryle ou aralkyle, et R⁷ et R⁸ représentent ensemble un groupe triméthylène ou tétraméthylène qui est éventuellement substitué par un groupe hydroxy, alcoxycarbonylamino ou acylamino, ou dans lequel un groupe -CH₂- est remplacé par -NH-, par un groupe -N(alcoxycarbonyl)-, -N(acyl)- ou par -S-, ou qui porte un cycle cycloalcane, aromatique ou hétéroaromatique soudé, et R⁹ représente un groupe alcoxycarbonyle, monoalkylcarbamoyle, monoaralkylcarbamoyle, monoarylcarbamoyle ou un groupe de formule 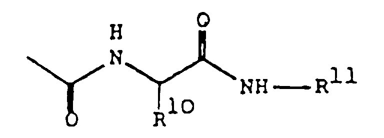 dans laquelle R¹⁰ et R¹¹ représentent chacun un groupe alkyle.

2. Alcools selon la revendication 1, dans lesquels R^{a} représente le groupe phtalimido.

3. Alcools selon la revendication 1 ou 2, dans lesquels R⁴ représente un groupe aralkyle.

4. Alcools selon l'une des revendications 1 à 3, dans lesquels R⁴ représente le groupe benzyle.

5. Alcools selon l'une des revendications 1 à 4, dans lesquels -N(R⁷)-CH(R⁸)(R⁹) représente un groupe de formule 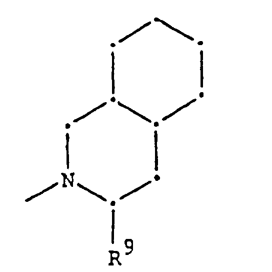 dans laquelle R⁹ représente un groupe monoalkylcarbamoyle.

6. Alcools selon la revendication 5, dans lesquels R⁹ représente le groupe tert-butylcarbamoyle.

7. Alcools selon l'une des revendications 1 à 5, dans lesquels R^{a} représente le groupe phtalimido, R⁴ représente le groupe benzyle et -N(R⁷)-CH(R⁸)(R⁹) représente un groupe de formule indiquée dans la revendication 5, dans lequel R⁹ représente le groupe tert-butylcarbamoyle.

8. N-tert-butyl-décahydro-2-[2(R)-hydroxy-4-phényl-3(S)-phtalimidobutyl]-(4aS,8aS)-isoquinoléine-3(S)-carboxamide.

9. 2-[3(S)-azido-2(R)-hydroxy-4-phénylbutyl)-N-tert-butyl-décahydro-(4aS,8aS)-isoquinoléine-3(S)-carboxamide.

10. Epoxydes de formule générale 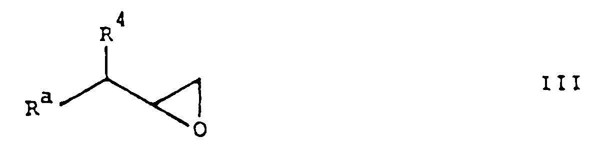 dans laquelle R^{a} représente le groupe azido ou phtalimido, et R⁴ représente un groupe alkyle, cycloalkyle, cycloalkylalkyle, aryle ou aralkyle, lorsque R⁴ est le groupe hexyle R^{a} ne pouvant pas être le groupe azido, et lorsque R⁴ est le groupe isobutyle R^{a} ne pouvant pas être le groupe phtalimido.

11. 2(S)-[2-phényl-1(S)-phtalimidoéthyl]oxiranne.

12. 2(S)-[1(S)-azido-2-phényléthyl]oxiranne.

13. Composés de formule générale 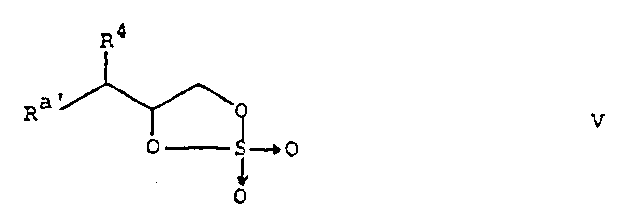 dans laquelle R^{a'} représente le groupe azido et R⁴ représente un groupe alkyle, cycloalkyle, cycloalkylalkyle, aryle ou aralkyle.

14. 2,2-dioxyde de 4(S)-[1(S)-azido-2-phényléthyl]-1,3,2-dioxothiolanne.

15. Procédé pour la préparation d'alcools selon l'une des revendications 1 à 9, caractérisé en ce que
(a) on fait réagir un époxide de formule générale 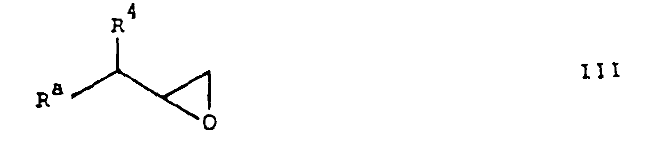 dans laquelle R^{a} et R⁴ ont les significations données dans la revendication 1,
avec un composé de formule générale 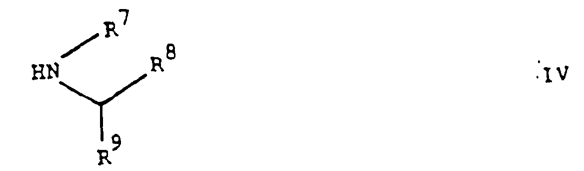 dans laquelle R⁷, R⁸ et R⁹ ont les significations données dans la revendication 1, ou
(b) pour la préparation d'un alcool de formule I dans lequel R^{a} représente le groupe azido, on fait réagir un composé de formule générale 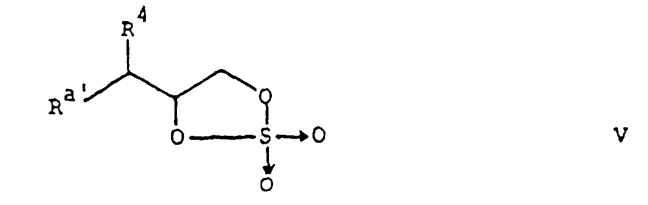 dans laquelle R^{a'} représente le groupe azido et R⁴ a la signification donnée dans la revendication 1,
avec un composé de formule IV ci-dessus.

16. Utilisation des alcools selon l'une des revendications 1 à 9, pour la préparation de dérivés d'aminoacides de formule générale 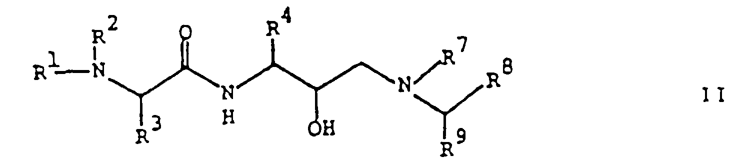 dans laquelle R¹ représente un groupe alcoxycarbonyle, aralcoxycarbonyle, alcanoyle, cycloalkylcarbonyle, aralcanoyle, aroyle, hétérocyclylcarbonyle, alkylsulfonyle, arylsulfonyle, monoaralkylcarbamoyle, cinnamoyle ou α-aralcoxycarbonylaminoalcanoyle, et R représente un atome d'hydrogène, ou R¹ et R forment ensemble, avec l'atome d'azote auquel ils sont liés, un groupe imido cyclique de formule 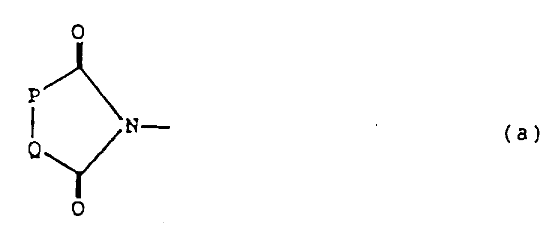 dans laquelle P et Q représentent ensemble un système aromatique, et R³ représente un groupe alkyle, cycloalkyle, aryle, aralkyle, hétérocyclylalkyle, cyanoalkyle, alkylthioalkyle, alkylsulfinylalkyle, carbamoylalkyle ou alcoxycarbonylalkyle, et R⁴, R⁷, R⁸ et R⁹ ont les significations données dans la revendication 1.

17. Utilisation selon la revendication 16 du N-tert-butyl-décahydro-2-[2(R)-hydroxy-4-phényl-3(S)-phtalimidobutyl]-(4aS,8aS)-isoquinoléine-3(S)-carboxamide pour la préparation du N-tert-butyl-décahydro-2-[2(R)-hydroxy-4-phényl-3(S)-[[N-(2-quinolylcarbonyl)-L-asparaginyl]amino]butyl]-(4aS,8aS)-isoquinoléine-3(S)-carboxamide.
